# EUROPÄISCHE PATENTSCHRIFT

(11) **EP 1 945 625 B1**
(45) Veröffentlichungstag und Bekanntmachung des Hinweises auf die Patenterteilung: **03.03.2010**
(21) Anmeldenummer: 06818364.9
(22) Anmeldetag: 03.11.2006
(51) Int. Cl.: C07D 311/78

(54) **PHOTOCHROME SPIRODIHYDROPHENANTHROPYRANE**
PHOTOCHROMIC SPIRODIHYDROPHENANTHROPYRANS
SPIRODIHYDROPHENANTHROPYRANES PHOTOCHROMES

(30) Priorität: 10.11.2005 DE 102005053986
(43) Veröffentlichungstag der Anmeldung: 23.07.2008
(73) Patentinhaber: Rodenstock GmbH, 80469 München (DE)
(72) Erfinder: WEIGAND, Udo, 80638 München (DE); MELZIG, Manfred, 82234 Wessling (DE); ROHLFING, Yven, 81547 München (DE)
(74) Vertreter: Müller-Boré & Partner Patentanwälte
(86) Internationale Anmeldenummer: PCT/EP2006/010562
(87) Internationale Veröffentlichungsnummer: WO 2007/054240

(56) Entgegenhaltungen:
- EP-A- 1 529 780
- WO-A-01/94336
- WO-A-02/22594

## Beschreibung

Die vorliegende Erfindung betrifft spezifische photochrome Spirodihydrophenanthropyrane sowie deren Verwendung in Kunststoffen aller Art, insbesondere für ophthalmische Zwecke. Es handelt sich bei den erfindungsgemäßen Verbindungen um photochrome Pyran-Verbindungen, die vom 9,10-Dihydrophenanthren abgeleitet sind, wobei mindestens einer der beiden Kohlenstoffatome in 9- oder 10-Position einem weiteren Ringsystem angehört und somit eine Spiro-Verknüpfungsstelle bildet.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, daß diese Farbstoffmoleküle durch Lichtenergie in einen angeregten Zustand übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, die in angeregter Form verschiedene Färbungen, wie Gelb, Orange oder Rotorange, zeigen.

Als weitere Verbindungsklasse photochromer Verbindungen sind höher annellierte Pyrane von Interesse, die aufgrund ihres größeren Ringsystems längerwellig absorbieren und rote, violette und blaue Farbtöne ergeben. Diese können entweder von den 2H-Naphtho[1,2-b]pyranen oder den 3H-Naphtho[2,1-b]pyranen abgeleitete Systeme sein, die durch Annellierung an der f-Seite aus den jeweiligen Naphthopyran-Systemen hervorgehen.

Diarylchromene, insbesondere Naphthopyrane oder heterozyklisch annellierte Benzopyrane, die in 6-Stellung des Benzopyrans mit einem Phenylring oder allgemeiner einem aromatischen oder heteroaromatischen Ring substituiert sind, welcher zusätzlich über die 5-Stellung des Benzopyrans über mindestens ein Kohlenstoffatom, Sauerstoffatom oder Stickstoffatom verbrückt ist, sind derzeit die vielversprechendsten photochromen Verbindungen.

Wird diese Verbrückung nur über ein Atom erzeugt, so ergibt sich ein an das Benzopyran annellierter Fünfring. Beispiele finden sich für ein Kohlenstoffatom in US 5,645,767, US 5,723,072 sowie US 5,955,520 und für ein Sauerstoffatom in US 6,018,059.

In US 5,723,072 kann zusätzlich an diesem Grundsystem ein un-, mono- oder disubstituierter heterozyklischer Ring an der g-, h-, i-, n-, o- oder p-Seite des Indenonaphthopyrans annelliert sein. Es werden demzufolge Indeno[1,2-f]naphtho[1,2-b]pyrane mit einer sehr großen Variationsbreite an möglichen Substituenten offenbart.

In WO 96/14596, WO 99/15518, US 5,645,767, WO 98/32037 und US 5,698,141 werden vom 2H-Naphtho[1,2-b]pyran abgeleitete photochrome indenoannellierte Naphthopyran-Farbstoffe, die sie enthaltenden Zusammensetzungen sowie ein Verfahren zu ihrer Herstellung offenbart. In US 5,698,141 kann zusätzlich an diesem Grundsystem ein un-, mono- oder disubstituierter heterozyklischer Ring an der g-, h-, i-, n-, o- oder p-Seite des Indenonaphthopyrans annelliert sein. Von der jeweils sehr umfangreichen Substituentenliste sind auch ganz spezielle Spiro-Verbindungen umfaßt, und zwar solche Systeme mit einer spiroheterozyklischen Gruppe, worin einschließlich des Spiroatoms an der 13-Position des Grundsystems ein 5- bis 8-gliedriger Ring, der stets zwei Sauerstoffatome enthält, vorhanden ist. Eine weitere Ausführungsform des Spiroringes findet sich in der japanischen Anmeldung 344762/2000.

Wird diese Verbindung über zwei Atome erzeugt, so ergibt sich ein annellierter Sechsring mit allein für C, O und N vielfältigen Möglichkeiten. Verbindungen mit C=O und N-R (Lactam-Brücke) werden in US 6,379,591 beschrieben. Verbindungen mit einer unsubstituierten CH₂-CH₂-Brücke sowie einem annellierten Heterocyclus in 7,8-Stellung des zugrundeliegenden Benzopyrans sind in US 6,426,023 offenbart.

US 6,506,538 beschreibt die carbocyclischen Analogverbindungen, bei denen die H-Atome in der Brücke durch OH, (C₁-C₆) Alkoxy oder zwei H-Atome an einem C-Atom durch =O ersetzt sein können.

Wird diese Verbindung durch drei Atome erzeugt, ergibt sich ein annellierter 7-Ring mit sehr vielen Variationsmöglichkeiten durch Einfügen von Heteroatomen. Verbindungen mit einer CH₂-CH₂-CH₂-Brücke sind in US 6,558,583 beschrieben. Auch hier können die H-Atome in der Brücke durch OH, (C₁- C₆)-Alkyl oder (C₁- C₆)-Alkoxy oder zwei Wasserstoffatome an einem C-Atom durch =O ersetzt sein. Sie absorbieren bei gleichem Substitutionsmuster kürzerwellig als die annellierten 6-Ringe.

US 2004/0094753 beschreibt sowohl Verbindungen mit 2- wie mit 3-atomiger Brücke. Die zweiatomige (Kohlenstoff-)Brücke ist dabei zusätzlich mit einem Carbo- bzw. Heterocyclus annelliert. Die dreiatomige Brücke enthält drei C-Atome oder zwei C-Atome und ein O-Atom ohne zusätzliche Annellierung. Beide Ringe können vielfältige Substituenten tragen.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Der vorliegenden Erfindung liegt daher die Aufgabe zugrunde, eine Klasse photochromer Verbindung bereitzustellen, die - beschränkt auf Systeme mit einer zweiatomigen Brücke - deutlich verbesserte Eigenschaften gegenüber den im Stand der Technik beschriebenen Strukturen besitzen sollen. Diese sind in der Kombination von langwelligem Absorptionsmaximum, hoher Eindunkelungsleistung, sehr schneller Aufhellreaktion und sehr guter Lichtbeständigkeit zu finden.

Diese Aufgabe wird durch die in den Ansprüchen gekennzeichneten Gegenstände gelöst.

Insbesondere werden photochrome Spirodihydrophenanthropyrane mit der allgemeinen Formel (I) bereitgestellt: 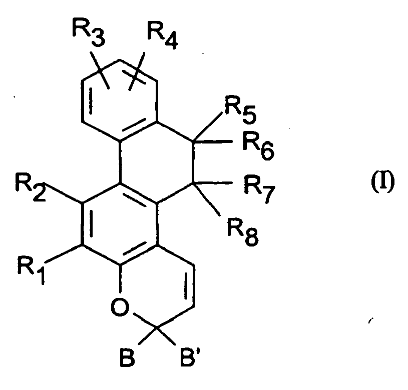 worin
die Reste R₁, R₂, R₃ und R₄ jeweils unabhängig voneinander einen Substituenten darstellen, ausgewählt aus der Gruppe α, bestehend aus einem Wasserstoffatom, einem (C₁ - C₆)-Alkylrest, einem (C₁- C₆)-Thioalkylrest, einem (C₃ - C₇)-Cycloalkylrest, der ein oder mehrere Heteroatome, wie beispielsweise O oder S, aufweisen kann, einem (C₁ - C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können; oder
die Reste R₁ und R₂ bzw. R₃ und R₄ jeweils unabhängig voneinander eine an den aromatischen Ring gebundene -A-(CH₂)ₖ-D- Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann; oder
die Reste R₁ und R₂ bzw. R₃ und R₄ jeweils unabhängig voneinander einen unsubstituierten, mono- oder disubstituierten, an die Phenanthren-Einheit annellierten Benzo- oder Pyridoring darstellen, dessen Substituenten aus der Gruppe α ausgewählt sein können;
die Reste R₅ und R₆ bzw. R₇ und R₈ jeweils unabhängig voneinander aus der Gruppe α ausgewählt sind,
mit der Maßgabe, daß mindestens entweder die Reste R₅ und R₆ oder die Reste R₇ und R₈ zusammen unter Einbeziehung des Spiro-Kohlenstoffatoms einen 3- bis 8-gliedrigen carbo- oder heteromonozyklischen Spiro-Ring, der gegebenenfalls einen oder mehrere Substituenten aus der Gruppe α trägt, bilden, an den ein bis drei aromatische oder heteroaromatische Ringsysteme annelliert sein können, wobei das Ringsystem unabhängig voneinander aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist, wobei zwei benachbarte annellierte Ringsysteme auch durch eine ortho,ortho'-Brücke miteinander verknüpft sein können, oder
mit der Maßgabe, daß mindestens entweder die Reste R₅ und R₆ oder die Reste R₇ und R₈ zusammen unter Einbeziehung des Spiro-Kohlenstoffatoms einen 7- bis 12-gliedrigen carbo-bizyklischen Spiro-Ring oder einen 7- bis 12-gliedrigen carbo-trizyklischen Spiro-Ring bilden, die jeweils gegebenenfalls ein oder mehrere Substituenten aus der Gruppe α tragen können,
B und B' unabhängig voneinander aus einer der folgenden Gruppen a), b) oder c) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist;
   wobei die Substituenten der Aryl- oder Heteroarylreste in a) und b) solche sind, ausgewählt aus der vorstehend definierten Gruppe α oder der Gruppe χ, bestehend aus Hydroxy, am Phenylring un-, mono- oder disubstituiertem 2-Phenylethenyl, am Phenylring un-, mono- oder disubstituiertem (Phenylimino)methylen, am Phenylring un-, mono- oder disubstituiertem (Phenylmethylen)imino, Amino, Mono-(C₁- C₆)-alkylamino, Di-(C₁- C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁ - C₆)-Alkyl, (C₁ - C₆)-Alkoxy, Brom, Chlor oder Fluor ausgewählt sein können;
   oder wobei zwei direkt benachbarte Substituenten eine Y-(CX₂)ₚ-Z-Gruppierung darstellen, wobei p = 1, 2 oder 3 ist, X Wasserstoff, CH₃ oder C₆H₅ sein kann und Y und Z unabhängig voneinander Sauerstoff, Schwefel, N-(C₁ - C₆)-Alkyl, N-C₆H₅, CH₂, C(CH₃)₂ oder C(C₆H₅)₂ sein können, wobei zwei oder mehrere benachbarte Kohlenstoffatome dieser Y-(CX₂)ₚ-Z-Gruppierung jeweils unabhängig voneinander auch Teil eines daran annellierten Benzo-Ringsystems sein können, welches jeweils wiederum einen oder mehrere Substituenten, ausgewählt aus der Gruppe α oder der Gruppe χ, aufweisen kann; oder
c) B und B' zusammen mit dem benachbarten Kohlenstoffatom des Pyran-Ringes einen un-, mono- oder disubstituierten 9,10-Dihydroanthracen-, Fluoren-, Thioxanthen-, Xanthen-, Benzo[b]fluoren-, 5H-Dibenzo[a,d]cyclohepten oder Dibenzosuberon-Rest oder einen gesättigten Kohlenwasserstoffrest, der (C₃ - C₁₂)-spiro-monozyklisch, (C₇ - C₁₂)-spiro-bizyklisch bzw. (C₇ - C₁₂)-spirc-trizyklisch ist, bilden, wobei die Substituenten der ungesättigten Cyclen unabhängig voneinander aus der Gruppe α bzw. der Gruppe χ ausgewählt sein können.

Die erfindungsgemäßen, von Spirodihydrophenanthropyranen abgeleiteten photochromen Verbindungen weisen im Vergleich zu derzeit im Stand der Technik verfügbaren Systemen mit einer zweiatomigen Brücke ein deutlich verbessertes Eigenschaftsprofil, insbesondere eine verbesserte Kombination von sehr guter Lebensdauer sowie schneller Aufhellungsgeschwindigkeit auf. Darüberhinaus zeigen die erfindungsgemäßen Verbindungen gegenüber solchen mit einer einatomigen Brücke neben einer schnelleren Aufhellungsgeschwindigkeit auch eine geringere Solvatochromie. Die erfindungsgemäßen Verbindungen zeigen eine ausgewogene Balance von langwelligem Absorptionsmaximum, hoher Eindunkelungsleistung, sehr schneller Aufhellreaktion und sehr guter Lichtbeständigkeit.

Bevorzugte photochrome Spirodihydrophenanthropyrane gemäß der vorliegenden Erfindung weisen die nachfolgende allgemeine Formel (II) auf: 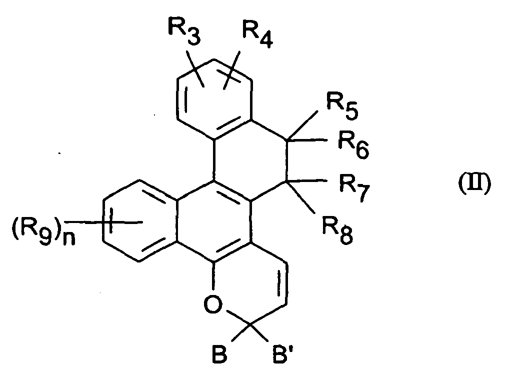 worin B, B', R₃, R₄, R₅, R₆, R₇ und R₈ wie vorgenannt definiert sind, R₉ aus der Gruppe α ausgewählt ist und n 0, 1, 2, 3 oder 4 ist.

Erfindungsgemäß bilden entweder die Reste R₅ und R₆ zusammen oder die Reste R₇ und R₈ zusammen unter Einbeziehung des Spiro-Kohlenstoffatoms einen 3- bis 8-gliedrigen carbo- oder heteromonozyklischen Spiro-Ring bzw. einen 7- bis 12- gliedrigen carbo-bizyklischen oder 7- bis 12-gliedrigen carbo-trizyklischen Spiro-Ring, wie vorstehend definiert. Es können aber auch sowohl die Reste R₅ und R₆ zusammen als auch die Reste R₇ und R₈ zusammen unter Einbeziehung des Spiro-Kohlenstoffatoms jeweils einen 3- bis 8-gliedrigen carbo- oder heteromonozyklischen Spiro-Ring bzw. einen 7- bis 12- gliedrigen carbo-bizyklischen oder 7- bis 12 gliedrigen carbo-trizyklischen Spiro-Ring bilden, so daß dann in 9- und 10-Stellung zwei spirozyklische Ringe vorliegen.

C₇ - C₁₂ spiro-bizyklische Systeme sind einem Fachmann wohlbekannt. Beispielhaft können hier Norbornan, Norbornen, 2,5-Norbornadien, Norcaran und Pinan genannt werden. Ein beispielhaftes C₇ - C₁₂ spiro-trizyklisches System ist Adamantan.

Vorzugsweise bilden die Reste R₇ und R₈ zusammen unter Einbeziehung des Spiro-Kohlenstoffatoms einen 3- bis 8-gliedrigen carbo- oder heterozyklischen Ring bilden, an den ein bis drei aromatische oder heteroaromatische Ringsysteme annelliert sein können, wobei das Ringsystem aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist. Dabei können auch zwei benachbarte annellierte Ringsysteme durch eine ortho,ortho'-Brücke, vorzugsweise eine Ethylen- oder eine 1,2-Ethendiyl-Brücke miteinander verknüpft sein, so daß beispielsweise im letzteren Fall folgende Struktureinheit vorliegt, d.h. eine Spiro-(phenanthren-4,5-diyl)-Einheit:

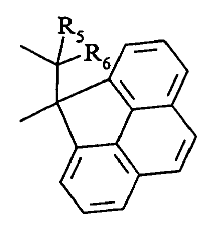

Wenn B bzw. B' für einen gesättigten Kohlenwasserstoffrest steht, der C₃ - C₁₂ spiro-monozyklisch, C₇ - C₁₂ spiro-bizyklisch oder C₇ - C₁₂ spiro-trizyklisch ist, so werden unter C₃ - C₁₂ spiro-monozyklisch ein dem Fachmann geläufiger 3-gliedriger bis 12-gliedriger Ring verstanden. Auch C₇ - C₁₂ spiro-bizyklische Systeme sind einem Fachmann wohlbekannt. Beispielhaft kann hier wiederum Norboman, Norbornen, 2,5-Norbomadien, Norcaran und Pinan genannt werden. Ein beispielhaftes C₇ - C₁₂ spiro-trizyklisches System ist Adamantan.

In einer bevorzugten Ausführungsform stellen die Reste R₇ und R₈ unter Einbeziehung des Spiro-Kohlenstoffatoms einen 5- bis 7-gliedrigen carbo-monozyklischen Ring dar, der wiederum einen, zwei, drei oder vier Substituenten, ausgewählt aus der Gruppe α, aufweisen kann, wobei an den carbozyklischen Ring ein bis drei Benzo-Ringe annelliert sein können, die wiederum einen oder zwei Substituenten, ausgewählt aus der Gruppe α, aufweisen können.

In einer anderen bevorzugten Ausführungsform stellen die Reste R₇ und R₈ unter Einbeziehung des Spiro-Kohlenstoffatoms einen 7- bis 8-gliedrigen carbo-bizyklischen Ring (d.h. ein Bicycloheptan- oder Bicyclooctan-Ringsystem) bzw. einen Adamantyl-Ring dar, die jeweils wiederum einen, zwei, drei oder vier Substituenten, ausgewählt aus der Gruppe α, aufweisen können.

In einer weiteren bevorzugten Ausführungsform sind die Reste B und B' unabhängig voneinander aus der Gruppe a), wie vorstehend definiert, ausgewählt.

Die Substituenten der Gruppe χ, welche Stickstoffatome aufweisen bzw. Amingruppen tragen, sind über diese an den Phenyl, Naphthyl- bzw. Phenanthrylrest der Gruppe a) gebunden.

Wenn bezüglich der Substituenten der Gruppe χ, welche an den Phenyl, Naphthyl- bzw. Phenanthrylrest der Gruppe a) für die Reste B bzw. B' gebunden sein können, zwei oder mehrere benachbarte Kohlenstoffatome dieser Y-(CX₂)ₚ-Z-Gruppierung jeweils unabhängig voneinander Teil eines daran annellierten Benzo-Ringsystems sein können, so bedeutet dies, dass dann die beiden Methylenkohlenstoffatome (- CH₂- CH₂ -) Teil eines annellierten Ringsystems werden. Wenn beispielsweise zwei oder drei Benzoringe annelliert sind, so können beispielsweise hier dann folgende Struktureinheiten vorliegen, wie nachstehend angeführt.

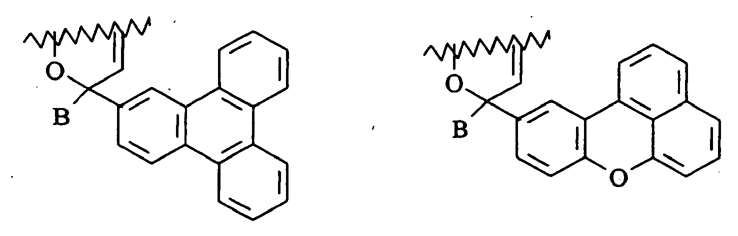

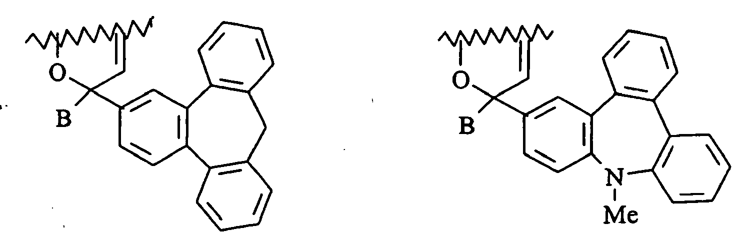

Selbstverständlich kann aber auch nur ein, über zwei benachbarte Kohlenstoffatome dieser Y-(CX₂)ₚ-Z-Gruppierung annellierter Benzoring vorliegen.

Die erfindungsgemäßen Verbindungen können in Kunststoffmaterialien bzw. Kunststoffgegenständen jeglicher Art und Form für eine Vielzahl von Einsatzzwekken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Spirodihydrophenanthropyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Spirodihydrophenanthropyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Spirodihydrophenanthropyrane durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Spirodihydrophenanthropyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

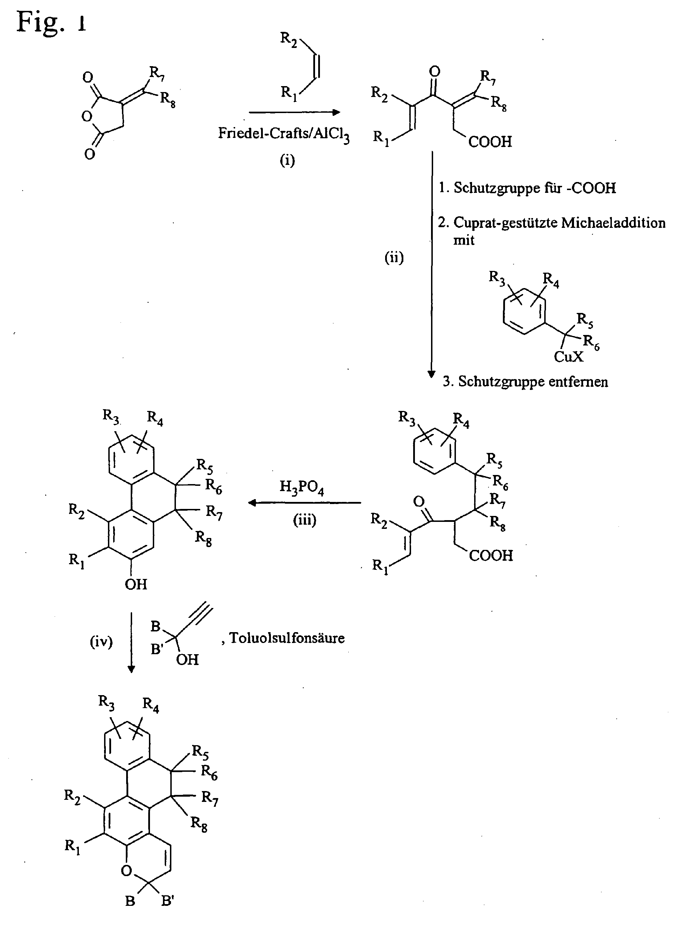

Die erfindungsgemäßen photochromen Verbindungen lassen sich durch Umsetzung von geeignet substituierten Spirodihydrophenanthren-Derivaten mit geeignet substituierten 2-Propin-1-ol-Derivaten in bekannter Weise (vgl. WO 02/22594) synthetisieren. Nachfolgend wird die Herstellung der erfindungsgemäßen Verbindungen anhand eines allgemeinen Reaktionsschemas beispielhaft erläutert (siehe Figur 1).

Geeignet substituierte Methylidensuccinanhydride werden in einem ersten Schritt einer Friedel-Crafts-Reaktion mit geeignet substituierten 1,2-Ethylenen unterworfen (Schritt (i)). Die -COOH Gruppe des daraus resultierenden Intermediats wird anschließend geschützt und dieses Intermediat einer Cuprat-gestützten Michael-Addition mit entsprechend substituierten Benzylderivaten unterworfen (Schritt (ii)). Nach Entfernung der Carbonsäure-Schutzgruppe werden via intramolekularer Cyclisierung mittels Phosphorsäure entsprechend substituierte Spirodihydrophenanthren-Derivate gebildet (Schritt (iii)). Anschließend werden diese substituierten Spirodihydrophenanthren-Derivate mit geeignet substituierten 2-Propin-1-ol-Derivaten gemäß Schritt (iv) zu den erfindungsgemäßen Verbindungen umgesetzt.

## Patentansprüche

1. Photochrome Spirodihydrophenanthropyrane mit der allgemeinen Formel (I): 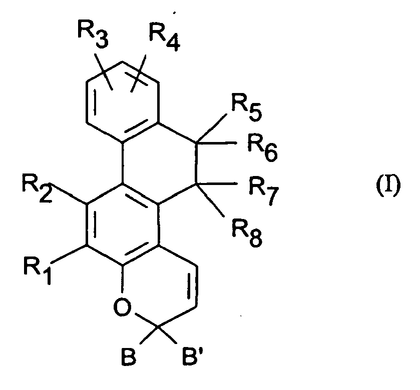 worin
die Reste R₁, R₂, R₃ und R₄ jeweils unabhängig voneinander einen Substituenten darstellen, ausgewählt aus der Gruppe α, bestehend aus einem Wasserstoffatom, einem (C₁ - C₆)-Alkylrest, einem (C₁ - C₆)-Thioalkylrest, einem (C₃ - C₇)-Cycloalkylrest, der ein oder mehrere Heteroatome, wie beispielsweise O oder S, aufweisen kann, einem (C₁ - C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können; oder
die Reste R₁ und R₂ bzw. R₃ und R₄ jeweils unabhängig voneinander eine an den aromatischen Ring gebundene -A-(CH₂)ₖ-D- Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann; oder
die Reste R₁ und R₂ bzw. R₃ und R₄ jeweils unabhängig voneinander einen unsubstituierten, mono- oder disubstituierten, an die Phenanthren-Einheit annellierten Benzo- oder Pyridoring darstellen, dessen Substituenten aus der Gruppe α ausgewählt sein können;
die Reste R₅ und R₆ bzw. R₇ und R₈ jeweils unabhängig voneinander aus der Gruppe α ausgewählt sind,
mit der Maßgabe, daß mindestens entweder die Reste R₅ und R₆ oder die Reste R₇ und R₈ zusammen unter Einbeziehung des Spiro-Kohlenstoffatoms einen 3- bis 8-gliedrigen carbo- oder heteromonozyklischen Spiro-Ring, der gegebenenfalls einen oder mehrere Substituenten aus der Gruppe α trägt, bilden, an den ein bis drei aromatische oder heteroaromatische Ringsysteme annelliert sein können, wobei das Ringsystem unabhängig voneinander aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist, wobei zwei benachbarte annellierte Ringsysteme auch durch eine ortho,ortho'-Brücke miteinander verknüpft sein können, oder
mit der Maßgabe, daß mindestens entweder die Reste R₅ und R₆ oder die Reste R₇ und R₈ zusammen unter Einbeziehung des Spiro-Kohlenstoffatoms einen 7- bis 12-gliedrigen carbo-bizyklischen Spiro-Ring oder einen 7- bis 12-gliedrigen carbo-trizyklischen Spiro-Ring bilden, die jeweils gegebenenfalls ein oder mehrere Substituenten aus der Gruppe α tragen können,
B und B' unabhängig voneinander aus einer der folgenden Gruppen a), b) oder c) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist;
wobei die Substituenten der Aryl- oder Heteroarylreste in a) und b) solche sind; ausgewählt aus der vorstehend definierten Gruppe α oder der Gruppe χ, bestehend aus Hydroxy, am Phenylring un-, mono- oder disubstituiertem 2-Phenylethenyl, am Phenylring un-, mono- oder disubstituiertem (Phenylimino)methylen, am Phenylring un-, mono- oder disubstituiertem (Phenylmethylen)imino, Amino, Mono-(C₁ - C₆)-alkylamino, Di-(C₁ - C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁ - C₆)-Alkyl, (C₁ - C₆)-Alkoxy, Brom, Chlor oder Fluor ausgewählt sein können;
oder wobei zwei direkt benachbarte Substituenten eine Y-(CX₂)ₚ-Z-Gruppierung darstellen, wobei p = 1, 2 oder 3 ist, X Wasserstoff, CH₃ oder C₆H₅ sein kann und Y und Z unabhängig voneinander Sauerstoff, Schwefel, N-(C₁ - C₆)-Alkyl, N-C₆H₅, CH₂, C(CH₃)₂ oder C(C₆H₅)₂ sein können, wobei zwei oder mehrere benachbarte Kohlenstoffatome dieser Y-(CX₂)ₚ-Z-Gruppierung jeweils unabhängig voneinander auch Teil eines daran annellierten Benzo-Ringsystems sein können, welches jeweils wiederum einen oder mehrere Substituenten, ausgewählt aus der Gruppe α oder der Gruppe χ, aufweisen kann; oder
c) B und B' zusammen mit dem benachbarten Kohlenstoffatom des Pyran-Ringes einen un-, mono- oder disubstituierten 9,10-Dihydroanthracen-, Fluoren-, Thioxanthen-, Xanthen-, Benzo[b]fluoren-, 5H-Dibenzo[a,d]cyclohepten oder Dibenzosuberon-Rest oder einen gesättigten Kohlenwasserstoffrest, der (C₃ - C₁₂)-spiro-monozyklisch, (C₇ - C₁₂)-spiro-bizyklisch bzw. (C₇- C₁₂)-spiro-trizyklisch ist, bilden, wobei die Substituenten der ungesättigten Cyclen unabhängig voneinander aus der Gruppe α bzw. der Gruppe χ ausgewählt sein können.

2. Photochrome Spirodihydrophenanthropyrane nach Anspruch 1, welche die allgemeine Formel (II) aufweisen: 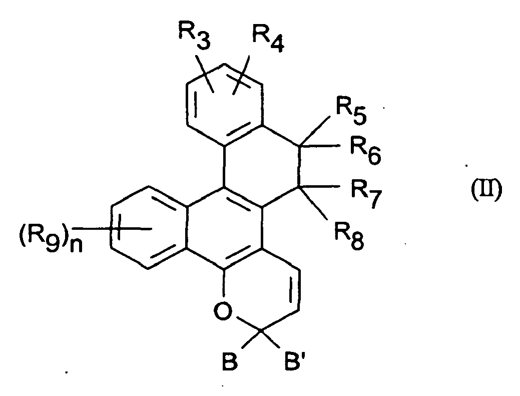 worin B, B', R₃, R₄, R₅, R₆, R₇ und R₈ wie vorgenannt definiert sind, R₉ aus der Gruppe α ausgewählt ist und n 0, 1, 2, 3 oder 4 ist.

3. Photochrome Spirodihydrophenanthropyrane nach Anspruch 1 oder 2, wobei die Reste R₇ und R₈ zusammen unter Einbeziehung des Spiro-Kohlenstoffatoms einen 3- bis 8-gliedrigen carbo- oder heterozyklischen Ring bilden, an den ein bis drei aromatische oder heteroaromatische Ringsysteme annelliert sein können,
wobei das Ringsystem aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist, wobei zwei benachbarte annellierte Ringsysteme auch durch eine ortho,ortho'-Brücke miteinander verknüpft sein können.

4. Photochrome Spirodihydrophenanthropyrane nach Anspruch 3, wobei die ortho,ortho'-Brücke eine Ethylen- oder eine 1,2-Ethendiyl-Brücke ist.

5. Photochrome Spirodihydrophenanthropyrane nach Anspruch 1 oder 2, wobei die Reste R₇ und R₈ unter Einbeziehung des Spiro-Kohlenstoffatoms einen 5- bis 7- gliedrigen carbo-monozyklischen Ring bilden, der wiederum einen, zwei, drei oder vier Substituenten, ausgewählt aus der Gruppe α, aufweisen kann, wobei an den carbozyklischen Ring ein bis drei Benzo-Ringe annelliert sein können, die wiederum einen oder zwei Substituenten, ausgewählt aus der Gruppe α, aufweisen können.

6. Photochrome Spirodihydrophenanthropyrane nach Anspruch 1 oder 2, wobei die Reste R₇ und R₈ unter Einbeziehung des Spiro-Kohlenstoffatoms einen 7- bis 8- gliedrigen carbo-bizyklischen Ring oder einen Adamantyl-Ring bilden, der jeweils wiederum einen, zwei, drei oder vier Substituenten, ausgewählt aus der Gruppe α, aufweisen kann.

7. Photochrome Spirodihydrophenanthropyrane nach einem der Ansprüche 1 bis 6, wobei B und B' unabhängig voneinander aus der Gruppe a) ausgewählt sind.

8. Verwendung der photochromen Spirodihydrophenanthropyrane nach einem der Ansprüche 1 bis 7 in und auf Kunststoffmaterialien.

9. Verwendung nach Anspruch 8, wobei das Kunststoffmaterial eine ophthalmische Linse ist.

## Claims

1. Photochromic spirodihydrophenanthropyrans with the general formula (I): 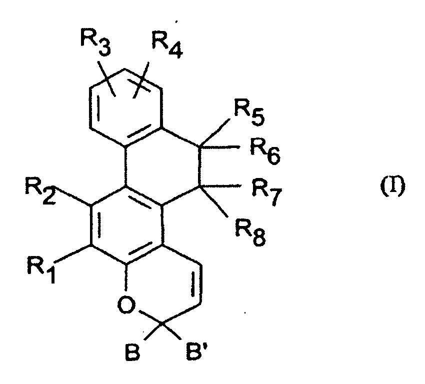 wherein
the radicals R₁, R₂, R₃ and R₄ represent respectively independently of each other substituents selected from the group α, comprising a hydrogen atom, a (C₁-C₆)alkyl radical, a (C₁-C₆)thioalkyl radical, a (C₃-C₇)cycloalkyl radical, which can have one or more heteroatoms, such as for example O or S, a (C₁-C₆)alkoxy radical, a hydroxy group, a trifluoromethyl group, bromine, chlorine, fluorine, an un-, mono- or disubstituted phenyl-, phenoxy-, benzyl-, benzyloxy-, naphthyl- or naphthoxy radical, the substituents being able in turn to be selected from the group α; or
the radicals R₁, R₂ or R₃ and R₄ form respectively independently of each other a -A-(CH₂)ₖ-D- group or -A-(C(CH₃)₂)ₖ-D- group with k = 1 or 2, A and D being selected independently of each other from oxygen, sulphur, CH₂, C(CH₃)₂ or C(C₆H₅)₂ and in turn a benzo ring being able to be anellated on this -A-(CH₂)ₖ-D- group; or
the radicals R₁ and R₂ or R₃ and R₄ represent respectively independently of each other an un-, mono- or disubstituted benzo- or pyrido ring which is anellated on the phenanthrene unit, the substituents of which ring can be selected from the group α;
the radicals R₅ and R₆ or R₇ and R₈ are selected respectively independently of each other from the group α,
with the proviso that at least either the radicals R₅ and R₆ or the radicals R₇ and R₈ together including the spiro carbon atom form a 3-to 8-member carbo- or heteromonocyclic spiro ring which carries possibly one or more substituents from the group α, on which ring one to three aromatic or heteroaromatic ring systems can be anellated, the ring system being selected independently of each other from the group β, comprising benzene, naphthalene, phenanthrene, pyridine, quinoline, furan, thiophene, pyrrole, benzofuran, benzothiophene, indole and carbazole, which in turn can be substituted with one or more substituents from the group α, two adjacent anellated ring systems also being able to be crosslinked with each other by means of an ortho,ortho' bridge,
with the proviso that at least either the radicals R₅ and R₆ or the radicals R₇ and R₈ together including the spiro carbon atom form a 7-to 12-member carbobicylic spiro ring or a 7- to 12-member carbotricyclic spiro ring which can carry possibly one or more substituents from the group α, or
B and B' are selected independently of each other from one of the following groups a), b) or c), wherein they
a) are mono-, di- and trisubstituted aryl radicals, the aryl radical being phenyl, naphthyl or phenanthryl;
b) are unsubstituted, mono- and disubstituted heteroaryl radicals, the heteroaryl radical being pyridyl, furanyl, benzofuranyl, thienyl, benzothienyl, 1,2,3,4-tetrahydrocarbazolyl or julolidinyl; the substituents of the aryl or heteroaryl radicals in a) and b) being those selected from the previously defined group α, or the group x comprising hydroxy, on the phenyl ring un-, mono- or disubstituted 2-phenylethenyl, on the phenyl ring un-, mono- or disubstituted (phenylimino)methylene, on the phenyl ring un-, mono- or disubstituted (phenylmethylene)imino, amino, mono-(C₁-C₆)alkylamino, di-(C₁-C₆)alkylamino, on the phenyl ring un-, mono- or disubstituted mono- and diphenylamino, piperidinyl, N-substituted piperazinyl, pyrrolidinyl, imidazolidinyl, pyrazolidinyl, indolinyl, morpholinyl, 2-6-dimethylmorpholinyl, thiomorpholinyl, azacycloheptyl, azacyclooctyl, un-, mono- or disubstituted phenothiazinyl, un-, mono- or disubstituted phenoxazinyl, un-, mono- or disubstituted 1,2,3,4-tetrahydroquinolinyl, un-, mono- or disubstituted 2,3-dihydro-1,4-benzoxazinyl, un-, mono- or disubstituted 1,2,3,4-tetrahydroisoquinolinyl, un-, mono- or disubstituted phenazinyl, un-, mono- or disubstituted carbazolyl, un-, mono- or disubstituted 1,2,3,4-tetrahydrocarbazolyl and un-, mono- or disubstituted 10,11-dihydrodibenz[b,f]azepinyl, the substituent or substituents being able to be selected independently of each other in turn from (C₁-C₆)alkyl, (C₁-C₆)alkoxy, bromine, chlorine or fluorine,
or two directly adjacent substituents representing a Y-(CX₂)ₚ-Z-grouping, wherein p = 1, 2 or 3, X being able to be hydrogen, CH₃ or C₆H₅, and Y and Z being able to be independently of each other oxygen, sulphur, N-(C₁-C₆)alkyl, N-C₆H₅, CH₂, C(CH₃)₂ or C(C₆H₅)₂, two or more adjacent carbon atoms of this Y-(CX₂)ₚ-Z-grouping being able to be independently of each other also part of a benzo ring system anellated thereon, which respectively in turn can have one or more substituents selected from the group α or the group x;
or
c) B and B', together with the adjacent carbon atom of the pyran ring, form an un-, mono- or disubstituted 9,10-dihydroanthracene-, fluorene-, thioxanthene-, xanthene-, benzo[b]fluorene-, 5H-dibenzo[a,d]cycloheptene- or dibenzosuberone radical or a saturated hydrocarbon radical which is (C₃-C₁₂)spiromonocylic, (C₇-C₁₂)spirobicylic or (C₇-C₁₂)spirotricylic, the substituents of the unsaturated cycles being able to be selected independently of each other from the group α or the group x.

2. Photochromic spirodihydrophenanthropyrans according to claim 1, which have the general formula (II): 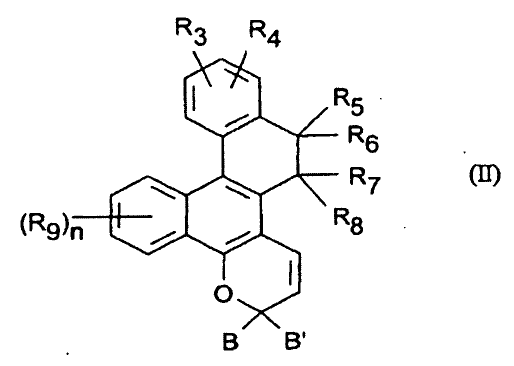 wherein B, B', R₃, R₄, R₅, R₆, R₇ and R₈ are defined as mentioned previously, R₆ is selected from the group α and n is 0, 1, 2, 3 or 4.

3. Photochromic spirodihydrophenanthropyrans according to claim 1 or 2, the radicals R₇ and R₈ together including the spiro carbon atom forming a 3- to 8-member carbo- or heterocyclic ring on which one to three aromatic or heteroaromatic ring systems can be anellated, the ring system being selected from the group β, comprising benzene, naphthalene, phenanthrene, pyridine, quinoline, furan, thiophene, pyrrole, benzofuran, benzothiophene, indole and carbazole, which in turn can be substituted with one or more substituents from the group α, two adjacent anellated ring systems also being able to be crosslinked with each other by means of an ortho,ortho' bridge.

4. Photochromic spirodihydrophenanthropyrans according to claim 3, the ortho,ortho'bridge being an ethylene or a 1,2-ethenediyl bridge.

5. Photochromic spirodihydrophenanthropyrans according to claim 1 or 2, the radicals R₇ and R₈ including the spiro carbon atom forming a 5- to 7-member carbomonocyclic ring, which in turn can have one, two, three or four substituents selected from the group α, one to three benzo rings being able to be anellated on the carbocyclic ring, which benzo rings in turn can have one or two substituents selected from the group α.

6. Photochromic spirodihydrophenanthropyrans according to claim 1 or 2, the radicals R₇ and R₈ including the spiro carbon atom forming a 7- to 8-member carbobicyclic ring or an adamantyl ring which respectively in turn can have one, two, three or four substituents selected from the group α.

7. Photochromic spirodihydrophenanthropyrans according to one of the claims 1 to 6, B and B' being selected independently of each other from the group a).

8. Use of the photochromic spirodihydrophenanthropyrans according to one of the claims 1 or 7 in and on plastic materials.

9. Use according to claim 8, the plastic material being an ophthalmic lens.

## Revendications

1. Spirodihydrophènanthropyranes photochromes de formule générale (I) : 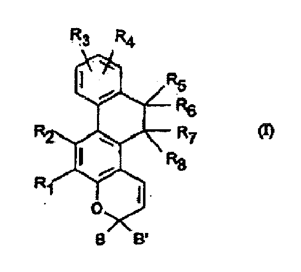 dans laquelle :
les radicaux R₁, R₂, R₃ et R₄ représentent, respectivement indépendamment l'un de l'autre, un substituant, choisi dans le groupe α, constitué par un atome d'hydrogène, un radical alkyle (en C₁ - C₆), un radical thioalkyle (en C₁ - C₆), un radical cycloalkyle (en C₃ - C₇), qui peut présenter un ou plusieurs hétéroatomes, comme par exemple O ou S, un radical alcoxy (en C₁ - C₆), un groupe hydroxy, un groupe trifluorométhyle, brome, chlore, fluor, un radical phényle, phénoxy, benzyle, benzyloxy, naphtyle ou naphtoxy non substitué, mono- ou disubstitué, où les substituants peuvent être de nouveau choisis dans le groupe α ; ou
les radicaux R₁ et R₂ ou R₃ et R₄ forment, respectivement indépendamment l'un de l'autre, un groupe -A-(CH₂)ₖ-D- ou -A-(C(CH₃)₂)ₖ-D- lié au cycle aromatique avec k = 1 ou 2, où A et D sont choisis, indépendamment l'un de l'autre, parmi l'oxygène, le soufre, CH₂, C(CH₃)₂ ou C(C₆H₅)₂, et où de nouveau un cycle benzénique peut être condensé à ce groupe -A-(CH₂)ₖ-D- ; ou
les radicaux R₁ et R₂ ou R₃ et R₄ représentent, respectivement indépendamment l'un de l'autre, un cycle benzénique ou pyridique, non substitué, mono- ou disubstitué, condensé au motif phénanthrène, dont les substituants peuvent être choisis dans le groupe α ;
les radicaux R₅ et R₆ ou R₇ et R₈ sont choisis, respectivement indépendamment l'un de l'autre, dans le groupe α,
à la condition qu'au moins soit les radicaux R₅ et R₆ soit les radicaux R₇ et R₈ forment, conjointement en intégrant l'atome de carbone spirannique, un cycle spiro carbo- ou hétéromonocyclique de 3 à 8 chaînons, qui porte le cas échéant un ou plusieurs substituants choisis dans le groupe α, auquel un à trois systèmes cycliques aromatiques ou hétéro-aromatiques peuvent être condensés, où le système cyclique est choisi, indépendamment, dans le groupe β constitué par le benzène, le naphtalène, le phénanthrène, la pyridine, la quinoline, le furane, le thiophène, le pyrrole, le benzofurane, le benzothiophène, l'indole et le carbazole, qui peuvent être à leur tour substitués par un ou plusieurs substituants choisis dans le groupe α, où deux systèmes cycliques condensés adjacents peuvent aussi être reliés l'un à l'autre par un pont ortho,ortho', ou
à la condition qu'au moins soit les radicaux R₅ et R₆ soit les radicaux R₇ et R₈ forment, conjointement en intégrant l'atome de carbone spirannique, un cycle spiro carbobicyclique de 7 à 12 chaînons ou un cycle spiro carbotricyclique de 7 à 12 chaînons, qui peuvent porter respectivement le cas échéant un ou plusieurs substituants choisis dans le groupe α,
B et B' sont choisis, indépendamment l'un de l'autre, dans l'un des groupes suivants a), b) ou c) :
a) les radicaux aryles mono-, di- et trisubstitués, où le radical aryle est phényle, naphtyle ou phénanthryle ;
b) les radicaux hétéroaryles non substitués, mono- et disubstitués, où le radical hétéroaryle est pyridyle, furanyle, benzofuranyle, thiényle, benzothiényle, 1,2,3,4-tétrahydrocarbazolyle ou julolidinyle ;
où les substituants des radicaux aryles ou hétéroaryles dans a) et b) sont ceux choisis dans le groupe α précédemment défini ou le groupe χ constitué par hydroxy, 2-phényléthényle non substitué, mono- ou disubstitué sur le cycle phényle, (phénylimino)méthylène non substitué, mono- ou disubstitué sur le cycle phényle, (phénylméthylène)imino non substitué, mono- ou disubstitué sur le cycle phényle, amino, mono-(C₁-C₆)alkylamino, di (C₁-C₆)-alkylamino, mono- et diphénylamino non substitué, mono- ou disubstitué sur le cycle phényle, pipéridinyle, pipérazinyle N-substitué, pyrrolidinyle, imidazolidinyle, pyrazolidinyle, indolinyle, morpholinyle, 2,6-diméthylmorpholinyle, thiomorpholinyle, azacyclo-heptyle, azacyclooctyle, phénothiazinyle non substitué, mono- ou disubstitué, phénoxazinyle non substitué, mono- ou disubstitué, 1,2,3,4-tétrahydroquinolinyle non substitué, mono- ou disubstitué, 2,3-dihydro-1,4-benzoxazinyle non substitué, mono- ou disubstitué, 1,2,3,4-tétrahydroisoquinolinyle non substitué, mono- ou disubstitué, phénazinyle non substitué, mono- ou disubstitué, carbazolyle non substitué, mono- ou disubstitué, 1,2,3,4-tétrahydrocarbazolyle non substitué, mono- ou disubstitué, et 10,11-dihydrodibenzo[b,f]azépinyle non substitué, mono- ou disubstitué, où le ou les substituants peuvent être choisis, indépendamment l'un de l'autre, de nouveau parmi alkyle (en C₁ - C₆), alcoxy (en C₁ - C₆), brome, chlore ou fluor ;
ou où deux substituants directement adjacents représentent un groupement Y-(CX₂)ₚ-Z-, où p = 1, 2 ou 3, X peut être hydrogène, CH₃ ou C₆H₅ et Y et Z peuvent être, indépendamment l'un de l'autre, oxygène, soufre N-(C₁-C₆) alkyle, N-C₆H₅, CH₂, C(CH₃) ou C(C₆H₅)₂, où deux atomes de carbone adjacents ou plus de ce groupement Y-(CX₂)ₚ-Z- peuvent, respectivement indépendamment l'un de l'autre, faire aussi partie d'un système benzocyclique qui y est condensé, qui peut présenter de nouveau respectivement un ou plusieurs substituants, choisis dans le groupe α, ou le groupe χ ;
ou
c) B et B' forment, conjointement avec l'atome de carbone adjacent du cycle pyrannique, un radical 9,10-dihydroanthracène, fluorène, thioxanthène, xanthène, benzo[b]fluorène, 5H-dibenzo[a,d]-cycloheptène ou dibenzosubérone non substitué, mono- ou disubstitué ou un radical hydrocarboné saturé, qui est spiro-monocyclique (en C₃-C₁₂), spiro-bicyclique (en C₇-C₁₂) ou spiro-tricyclique (en C₇-C₁₂), où les substituants des cycles insaturés peuvent être choisis, indépendamment l'un de l'autre, dans le groupe α ou le groupe χ.

2. Spirodihydrophènanthropyranes photochromes selon la revendication 1, qui présentent la formule générale (II) : 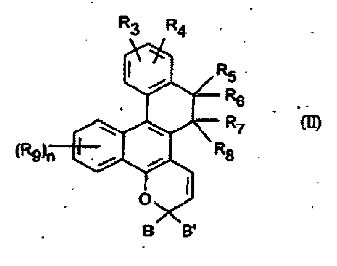 dans laquelle B, B', R₃, R₄, R₅, R₆, R₇ et R₈ sont tels que définis précédemment, R₉ est choisi dans le groupe α et n est égal à 0, 1, 2, 3 ou 4.

3. Spirodihydrophènanthropyranes photochromes selon la revendication 1 ou 2, où les radicaux R₇ et R₈ forment, conjointement en intégrant l'atome de carbone spirannique, un cycle carbo- ou hétérocyclique de 3 à 8 chaînons, auquel un à trois systèmes cycliques aromatiques ou hétéro-aromatiques peuvent être condensés, où le système cyclique est choisi dans le groupe β constitué par le benzène, le naphtalène, le phénanthrène, la pyridine, la quinoline, le furane, le thiophène, le pyrrole, le benzofurane, le benzothiophène, l'indole et le carbazole, qui peuvent être à leur tour substitués par un ou plusieurs substituants choisis dans le groupe α, où deux systèmes cycliques condensés adjacents peuvent aussi être reliés l'un à l'autre par un pont ortho,ortho'.

4. Spirodihydrophènanthropyranes photochromes selon la revendication 3, où le pont ortho,ortho' est un pont éthylène ou un pont 1,2-éthènediyle.

5. Spirodihydrophènanthropyranes photochromes selon la revendication 1 ou 2, où les radicaux R₇ et R₈ forment, en intégrant l'atome de carbone spirannique, un cycle carbo-monocyclique de 5 à 7 chaînons, qui peut présenter de nouveau un, deux, trois ou quatre substituants, choisis dans le groupe α, où un à trois cycles benzéniques, qui peuvent être à leur tour substitués par un ou deux substituants choisis dans le groupe α, peuvent être condensés au cycle carbocyclique.

6. Spirodihydrophènanthropyranes photochromes selon la revendication 1 ou 2, où les radicaux R₇ et R₈ forment, en intégrant l'atome de carbone spirannique, un cycle carbo-bicyclique de 7 à 8 chaînons ou un cycle adamantyle, qui peut présenter de nouveau respectivement un, deux, trois ou quatre substituants, choisis dans le groupe α.

7. Spirodihydrophènanthropyranes photochromes selon l'une des revendications 1 à 6, où B et B' sont choisis, indépendamment l'un de l'autre dans le groupe a) .

8. Utilisation des spirodihydrophènanthropyranes photochromes selon l'une des revendications 1 à 7 dans et sur des matériaux plastiques.

9. Utilisation selon la revendication 8, où le matériau plastique est une lentille ophtalmique.
